# EUROPEAN PATENT SPECIFICATION

(11) **EP 0 261 927 B1**
(45) Date of publication and mention of the grant of the patent: **18.11.1993**
(21) Application number: 87308374.5
(22) Date of filing: 22.09.1987
(51) Int. Cl.: A61B 5/02

(54) **Patient monitoring system having transportable data module and display unit**
Patientenüberwachungseinrichtung mit transportierbaren Datenmodule und Anzeigegerät
Dispositif de surveillance de malade comportant un module de données transportable et une unité de présentation

(30) Priority: 26.09.1986 US 912359
(43) Date of publication of application: 30.03.1988
(73) Proprietor: MARQUETTE ELECTRONICS, INC., Milwaukee Wisconsin 53223 (US)
(72) Inventor: Cudahy, Michael J., Delray Beach Florida 33444 (US); Huerga, Carlos De La, Shorewood Wisconsin 53211 (US); Arneson, Harold N., Waukesha Wisconsin 53186 (US); Divers, R. Thomas, Mequon Wisconsin 53092 (US); Altman, Barry J., Mequon Wisconsin 53092 (US)
(74) Representative: Brunner, Michael John

(56) References cited:
- US-A- 4 051 522
- US-A- 4 216 462
- US-A- 4 378 021
- US-A- 4 546 436

## Description

The present invention relates to a system for providing continuous physiological condition monitoring of a patient, including periods during which the patient is in transport from one location to another.

In a hospital or other healthcare setting, it is frequently necessary to observe critical physiological conditions of a patient, such as temperature, breath rate, pulse, blood pressure, ECG data, and cardiac output. Other conditions may be observed, depending on the injury or illness of the patient.

The physiological condition data is obtained by sensors applied to the patient. The sensors are connected to a monitor by cables, the monitor usually being mounted beside the patient's bed. Such monitors maybe energized by mains power and display the physiological condition data in graphic and/or alpha-numeric form for observation. The monitor is often connected to a central system for recording the data.

It is usually necessary or desirable to continuously observe the patient's physiological condition in order to detect the onset of changes. However, due to the stationary nature of a bedside or surgical monitor, it is difficult to maintain continuous surviellance when the patient must be moved, for example, from the operating room to the recovery room, from the recovery room to the intensive care unit, or from the intensive care unit to his/her hospital room. It is possible to use another monitor at these times having a battery power supply. However, this requires disconnection of the stationary monitor and connection of the additional monitor at the beginning of transport and the reverse procedure at the end of transport. This is time consuming; provides the possibility for error; loses set up data, scalars, trend data, alarm limits and the like; and may interrupt continuous monitoring as the sensors are connected and disconnected. The additional monitor will usually not be compatible with the stationary monitor or central data processing system, causing problems in handling of the physiological condition data.

For the foregoing reasons, patients may not be monitored adequately, or at all, for a critical physiological condition at certain times, such as during transport.

A portable system has been proposed, see US-A-4546436, in which the data collection unit is portable and removably connectable to a non-portable base station to provide for display of data, but does not provide for continuous uninterrupted monitoring of a patient, for example, when the patient is in transport.

It is, therefore, an object of the present invention to provide a physiological condition monitoring system that can provide continuous, uninterrupted, monitoring of a patient, including periods when the patient is in transport.

According to the present invention there is provided a monitoring system for providing continuous, uninterrupted, monitoring of one or more physiological conditions of a patient, including periods of transport of the patient from one location to another, in use the patient having physiological condition data sensors applied thereto; the system comprising:
a substantially non-transportable, first display unit providing a data display of the physiological conditions being monitored;
a data acquisition and processing module for receiving data from the sensors; the system being being characterised by
a separate portable, second display unit capable of accompanying the patient during transport and providing a data display of the physiological conditions being monitored; and
first and second connectors for coupling the module simultaneously to the first and second display units or coupling the module to either of the display units individually, whereby the module may drive the display units to display data of the physiological conditions being monitored.

The monitoring system of the present invention is simple and easy to use and avoids the multiple connection and disconnection of sensors heretofore required. It enables maintenance of all setup data, alarm limits, scalars, and the like during monitoring of the patients.

The invention also includes a portable monitoring system for displaying physiological condition data of a patient to which physiological condition data sensors are applied, the system comprising:
a data acquisition and processing module for receiving data from the sensors; the module having means for connecting the module to a display unit which is not intended for transport; and being characterised by
a portable display unit having a base member providing a compartment and a screen for displaying physiological condition data; and
the data acquisition and processing module being insertable in the compartment of the base member of the portable unit, the module having
a first connector for connecting the module to the portable unit so that the module may drive the screen to display physiological condition data; and wherein said means is a second connector.

In use, the data acquisition and processing module is typically received in the non-transportable unit when the patient is, for example, in the recovery room. When the patient is to be moved, the module is additionally connected to the portable, second display unit so that physiological condition data is displayed on both units. The module is then removed from the non-transportable display unit and placed in the portable display unit to accompany the patient during transport. At the completion of the transport, the operative sequence is reversed.

One example of a system according to the invention will now be described with reference to the accompanying drawings, in which:-
Fig. 1 shows the monitoring system when its data acquisition and processing module is received in the generally non-transportable monitor;
Fig. 2 shows the module and the manner of inserting same into the portable display unit;
Fig. 3 shows the monitoring system when the data acquisition and processing module is mounted in the portable display unit; and,
Fig. 4 is a fragmentary view of a portion of the module and non-transportable display unit showing a connector on the module and a corresponding connector on the non-transportable display unit.

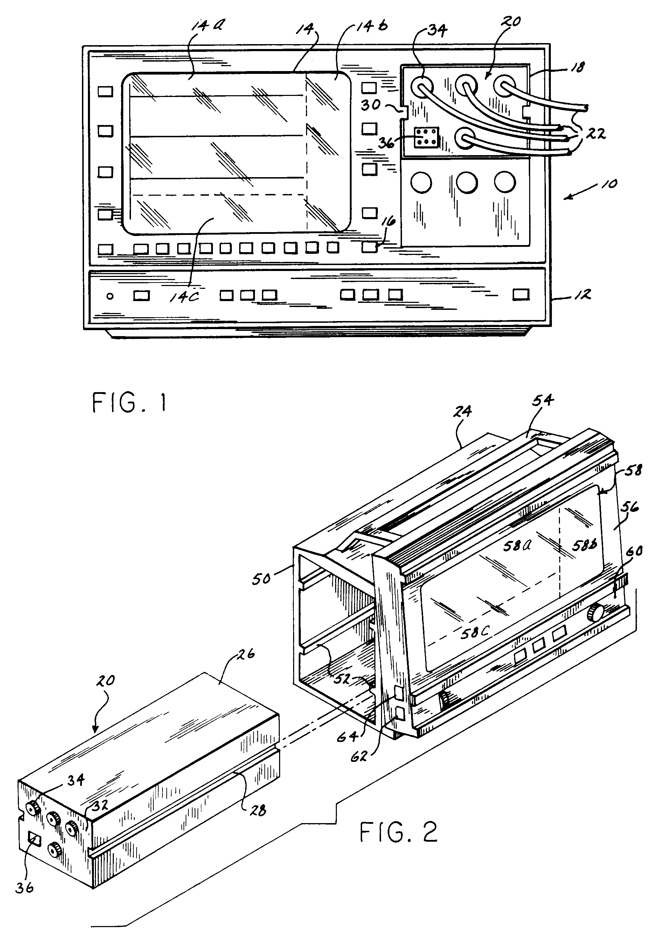

Fig. 1 shows a generally non-transportable monitor or display unit 10 which is typically mounted on the wall at a patient's bed in an operating room, recovery room, intensive care unit, hospital room or other care facility. The unit is energized by a power cord, not shown, from conventional electrical mains power. The display unit 10 includes a housing 12 having a screen 14 on which physiological data can be displayed in graphic or alpha-numeric form in a predetermined manner. Typically, the screen will include a portion 14a graphically displaying a plurality of physiological data as waveforms, a portion 14b displaying certain values such as heart rate, blood pressure, and temperature in numerical form, and a menu portion 14c in which commands, data requests, and the like are displayed. The display unit 10 contains a microprocessor or other circuitry for operating the monitor and for driving the screen 14. A plurality of control elements, such as a button 16, control the circuitry in the display unit 10 to control the operation of the monitor, the display of physiological condition data, the setting of alarm limits for such conditions, and the like. There may be provided in the display unit 10 an audible alarm that sounds when set limits are exceeded. The display unit 10 contains a recess 18 for receiving a data acquisition and processing module 20.

The data acquisition and processing module 20 accepts inputs from sensor cables 22 connected to the patient, processes the input data to derive waveforms and values therefrom, and provides a physiological condition data signal output for driving the display of either the non-transportable display unit 10 or a separate portable display unit 24, hereinafter described. The module 20 also stores data, such as alarm limits, obtained from the control element of the display unit.

The module 20 has a housing 26 for containing acquisition and processing circuity of conventional construction necessary to obtain the physiological condition data signal output, establish alarm limits, and the like. The housing 26 also includes a battery for powering the circuitry, typically of the rechargeable lead-acid type. The housing 26 has grooves 28 that assist in positioning the module in the display unit, the grooves 28 mating with ridges 30 of the recess 18 in the non-transportable display unit 10.

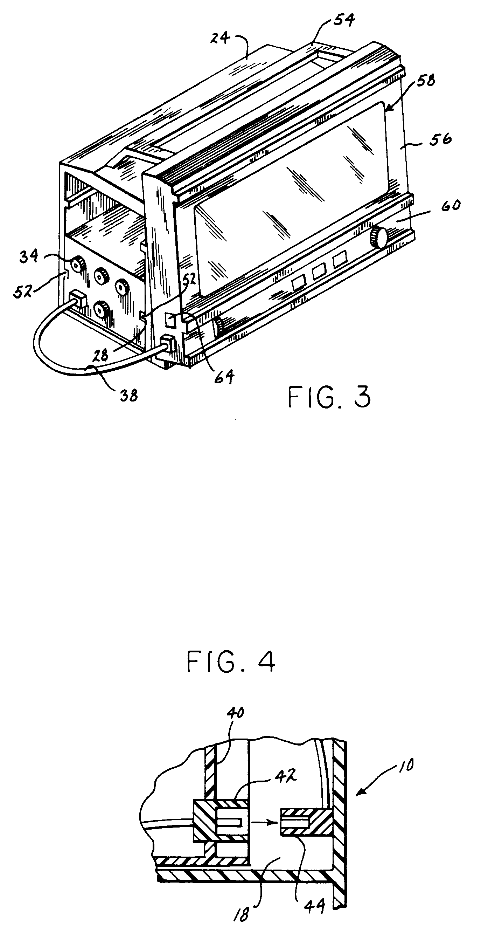

The front panel 32 of the module 20 contains input connectors 34 for receiving sensor cables 22 and may also contain a battery charge indicator for indicating when the battery is being recharged. The front panel 32 has a jack socket 36 for a cord 38 for connecting the module 20 to a portable display unit 24, in the manner shown in Fig. 3, and may also contain output jacks that provide direct outputs of desired data, such as ECG or blood pressure, to recording writers, or other equipment.

The rear panel 40 of the module 20, as shown in Fig. 4, contains a pin type connector 42 that mates with a corresponding connector 44 in the back of the recess 18 of the display unit 10. Connectors 42 and 44 supply power and data to, and receive data from, the module 20, when the module is inserted in the recess 18. Connectors 42, 44 may also supply power to a battery charging circuit in the module 20.

The portable display unit 24 includes a base member which has a rectangular tubular configuration closed by a vertical wall at one end, i.e., the right-hand end, as shown in Figs. 2 and 3, to form a compartment for receiving the data acquisition and processing module 20, in the manner shown in Fig. 3. Guide rails 52 on the inside of the base member 50 mate with the grooves 28 on the module 20 to assist in positioning the module in the compartment of the base member 50. The portable display unit 24 is shown in Figs. 2 and 3 with a compartment large enough to receive another module, such as one containing a writer for recording physiological condition data, if desired. Alternatively, the portable display unit 24 may be formed so that the compartment is sized to receive only the module 20.

A handle 54 is fastened to the base member 50 so that the portable display unit 24 and the module or modules in the compartment of the base member 50 may be lifted or moved.

A screen element 56 is mounted on one side of the base member 50 and is of the "flat panel" type or configuration. The screen element 56 includes a screen 58 which may be of the liquid crystal display type or may be of electroluminescent, plasma or gas discharge, or other type. The screen 58 may be operated and controlled by a microprocessor or other circuitry in the portable display unit 24. The format of the screen 58 resembles that of the screen 14 and displays the same physiological condition data. The screen 58 thus includes a portion 58a graphically displaying the physiological condition data as waveforms, a portion 58b displaying values in alpha-numeric form, and a menu portion 58c. The screen member 56 also contains a control panel 60 for the screen 58 and the data acquisition and processing module 20. Typical controls include a contrast control for the screen 58, an alarm reset control to silence an audio alarm in the display unit, a freeze control to halt waveform movement, a blood pressure zero control, and a control for accessing and displaying menus used to operate the monitor, for example to set limit values for the physiological conditions being monitored. The control action provided by the portable display unit 24 thus generally corresponds to that of the non-transportable display unit 10.

The portable display unit 24 also includes a connection jack socket 62 for the card 38. One or more auxiliary connection jacks 64 may be provided for peripheral equipment. Such peripheral equipment may include a printer or a remote control cable that permits accessing and operating the portable display unit 24 from a remote location.

Typical usage of the monitoring system of the present invention is described below in connection with a patient on a wheeled stretcher or gurney in a surgical recovery room. The module 20 is located in the recess 18 of display unit 10, and is energized through connectors 42 and 44. The sensors of cables 22 are applied to the patient and the cables 22 are inserted in the appropriate connectors 34 on the front panel 32 of the module 20. The controls 16 of the monitor 10 are operated to provide set-up data, such as alarm limits, calibration data, etc. for storage in the module 20.

The module 20 receives data via cables 22 and provides signals through connectors 42, 44 for driving the display 14 so that the physiological condition data may be observed by attending medical and nursing personnel.

When it is desired to transport the patient, one end of the cord 38 is connected into the jack socket 36 of the module 20 and the other end is connected into the jack socket 62 of the portable display unit 24. The controls of the control panel 60 are manipulated to turn on the display unit 24 so that the physiological condition data appears on the screen 58 and the module 20 thus drives the screen 58 of the portable display unit 24 in addition to the screen 14 of the display unit 10.

The module 20 is then removed from the recess 18 of the display unit 10 and placed in the compartment of the base member 50 of the portable display unit 24, in the manner shown in Fig. 2. The module 20 is powered by its internal storage battery. The display unit 10 is turned off.

The portable display unit 24 containing the module 20 may then be placed on the patient's stretcher for transport with the patient to his/her hospital room. Monitoring of the patient's physiological condition is obtained by means of the screen 58 during transport. In the hospital room, the patient is removed from the stretcher and placed on his/her bed. The portable display unit 24 is similarly moved so that monitoring is continued.

If long term monitoring is desired, a non-transportable display unit 10, similar to that described above, proximate to the patient's bed may be employed, the module 20 being removed from the base member 50 of the display unit 24 and placed in a recess 18 of the new display unit 10. The cord 38 remains connected between the module 20 and the display unit 24. The display unit 10 is then turned on so that the module 20 provides physiological condition data on the screen 14 as well as on the screen 58. Thereafter, the screen 58 may be turned off and the cord 38 removed from the jack sockets 36 and 62. Further monitoring is carried out on the display unit 10.

While module 20 is described above as placed in a compartment in the portable display unit 24, it will be appreciated that this is not necessary. For example, the module 20 may be placed near the patient and display unit 24, without the module, placed on a shelf for greater visibility, if desired. The module 20 and the display unit 24 remain connected by the cord 38. Also while the patient monitoring system of the present invention has been described in a hospital setting, it will be appreciated that it may be used in other settings, such as ambulance or aircraft transport.

From the foregoing description, it will be apparent that the monitoring system of the present invention is capable of providing continuous monitoring of a patient, including periods when the patient is being transported. This is accomplished by the ability of the monitoring system of the present invention to drive both display units 10 and 24 simultaneously, when needed. It is also accomplished by the ability of the module 20 to be removed from the non-transportable display unit 10 and placed in, or in proximity to, a separate transportable display unit 24, and vice versa.

## Claims

1. A monitoring system for providing continuous, uninterrupted, monitoring of one or more physiological conditions of a patient, including periods of transport of the patient from one location to another, in use the patient having physiological condition data sensors applied thereto; the system comprising:
a substantially non-transportable, first display unit (10) providing a data display of the physiological conditions being monitored;
a data acquisition and processing module (20) for receiving data from the sensors; the system being characterised by
a separate portable, second display unit (24) capable of accompanying the patient during transport and providing a data display of the physiological conditions being monitored; and
first and second connectors (42,44; 36,38,62) for coupling the module simultaneously to the first and second display units or coupling the module to either of the display units individually, whereby the module (20) may drive the display units (10,24) to display data of the physiological conditions being monitored.

2. A monitoring system according to claim 1, wherein the first (10) and second (24) display units each include means (18,50) for receiving the module (20), and wherein the module is insertable in one or the other of the display units.

3. A portable monitoring system (20,24) for displaying physiological condition data of a patient to which physiological condition data sensors are applied, the system comprising:
a data acquisition and processing module (20) for receiving data from the sensors; the module having means (42) for connecting the module (20) to a display unit (10) which is not intended for transport; and being characterised by
a portable display unit (24) having a base member (50) providing a compartment and a screen (56) for displaying physiological condition data; and
the data acquisition and processing module (20) being insertable in the compartment of the base member (50) of the portable unit (24), the module having
a first connector (36,38) for connecting the module to the portable unit so that the module may drive the screen (56) to display physiological condition data; and
wherein said means (42) is a second connector.

4. A monitoring system according to any of claims 1 to 3, wherein the second connector (36,38) includes a connection cord (38) for coupling the module to the portable display unit (24).

5. A monitoring system according to claim 2 or claim 4 when dependent on claim 2, wherein the first connector includes means (42) for connecting the module (20) to the first display unit (10) when the module is inserted therein.

6. A monitoring system according to any of claims 1 to 5, wherein the module (20) contains a power supply for powering the module and the portable display unit (24) when the module is coupled thereto.

7. A monitoring system according to claim 1 or any claim when dependent theron, wherein the first display unit (10) is couplable to electric mains power and wherein the first display unit and the module (20) contain connection means for powering the module when the module is coupled to the first display unit.

8. A monitoring system according to claim 2 or claim 3, wherein the portable display unit (24) is formed to receive an auxiliary data acquisition and processing module.

9. A monitoring system according to any of claims 1 to 8, wherein the portable display unit (24) incorporates a flat panel display (56).

10. A monitoring system according to claim 1 or any claim when dependent thereon, further including a third, substantially non-transportable, display unit, and wherein the connectors are further capable of coupling the module (20) simultaneously to the portable display unit (24) and to one of the first and third display units (10) or coupling the module to one of the display units individually.

11. A monitoring system according to claim 1 or any claim when dependent theron, wherein the portable display unit (24) is capable of displaying data in a manner corresponding to that of the first display unit (10).

## Patentansprüche

1. Überwachungssystem zur kontinuierlichen, ununterbrochenen Überwachung eines oder mehrerer physiologischer Zustände eines Patienten - auch während der Zeitperioden des Patiententransports von einem Ort zum anderen -, wobei im Betriebszustand am Patient Sensoren zur Erfassung physiologischer Zustandsdaten angelegt sind, und wobei das System umfaßt:
eine im wesentlichen nicht transportierbare erste Anzeigeeinheit (10), die eine patenanzeige der überwachten physiologischen Zustände liefert;
ein Datenerfassungs- und Verarbeitungsmodul (20) zum Empfang der Daten von den Sensoren, wobei das System gekennzeichnet ist durch
eine separate tragbare, zweite Anzeigeeinheit (24), welche den Patienten während eines Transportes begleiten kann und eine Datenanzeige der überwachten physiologischen Zustände liefern kann; und
erste und zweite Steckverbindungen (42,44; 36,38,62) zur gleichzeitigen Kopplung bzw. Verbindung des Moduls mit der ersten und zweiten Anzeigeeinheit oder zur individuellen Kopplung des Moduls mit einer der Anzeigeeinheiten, wobei das Modul (20) die Anzeigeeinheiten (10,24) be- bzw. antreiben kann, um die Daten der überwachten physiologischen Zustände anzuzeigen.

2. Überwachungssystem nach Anspruch 1, dadurch gekennzeichnet, daß die erste (10) und zweite (24) Anzeigeeinheit jeweils Mittel (18, 50) umfaßt zur Aufnahme des Moduls (20) und daß das Modul in der einen oder der anderen der Anzeigeeinheiten einführbar ist.

3. Tragbares Überwachungssystem (20, 24) für die Anzeige von physiologischen Zustandsdaten eines Patienten, an dem Sensoren zur Erfassung physiologischer Zustandsdaten anliegen, wobei das System enthält:
ein Datenerfassungs- und Verarbeitungsmodul (20) zum Empfang von Daten von den Sensoren, wobei das Modul ein Mittel (42) aufweist zur Verbindung des Moduls (20) mit einer Anzeigeeinheit (10), die nicht für einen Transport vorgesehen ist, und gekennzeichnet ist durch
eine tragbare Anzeigeeinheit (24) mit einem Grundglied (50), das ein Fach bzw. Aufnahmefach und einen Bildschirm (56) zur Anzeige von physiologischen Zustandsdaten zur Verfügung stellt; und
das Datenerfassungs- und Verarbeitungsmodul (20), das in das Fach des Grundgliedes (50) der tragbaren Einheit (24) einführ ist, wobei das Modul
eine erste Steckverbindung (36,38) zur Verbindung des Moduls mit der tragbaren Einheit aufweist, so daß das Modul den Bildschirm (56) zur Anzeige physiologischer Zustandsdaten antreiben kann, und wobei das Mittel (42) eine zweite Steckverbindung ist.

4. Überwachungssystem nach einem der Ansprüche 1-3, dadurch gekennzeichnet, daß die zweite Steckverbindung (36, 38) eine Leitungsschnur (38) umfaßt zur Kopplung des Moduls mit der tragbaren Anzeigeeinheit (24).

5. Überwachungssystem nach Anspruch 2 oder Anspruch 4 soweit rückbezogen auf Anspruch 2, dadurch gekennzeichnet, daß die erste Steckverbindung Mittel (42) zur Verbindung des Moduls (20) mit der ersten Anzeigeeinheit (10) enthält, wenn das Modul darin eingefügt ist.

6. Überwachungssystem nach einem der Ansprüche 1-5, dadurch gekennzeichnet, daß das Modul (20) eine Stromversorgung enthält zur Versorgung des Moduls und der tragbaren Anzeigeeinheit (24), wenn das Modul mit dieser gekoppelt ist.

7. Überwachungssystem nach Anspruch 1 oder irgendeinem darauf rückbezogenen Anspruch, dadurch gekennzeichnet, daß die erste Anzeigeeinheit (10) an die elektrische Netzversorgung anschließbar ist und daß die erste Anzeigeeinheit und das Modul (20) Verbindungsmittel enthalten zur Versorgung des Moduls, wenn das Modul mit der ersten Anzeigeeinheit gekoppelt ist.

8. Überwachungssystem nach Anspruch 2 oder 3, dadurch gekennzeichnet, daß die tragbare Anzeigeeinheit (24) zur Aufnahme eines Hilfsdatenerfassungs- und Verarbeitungsmoduls ausgebildet ist.

9. Überwachungssystem nach einem der Ansprüche 1-8, dadurch gekennzeichnet, daß die tragbare Anzeigeeinheit (24) eine Flachbildanzeige (56) umfaßt.

10. Überwachungssystem nach Anspruch 1 oder irgendeinem darauf rückbezogenen Anspruch, dadurch gekennzeichnet, daß das Überwachungssystem des weiteren eine dritte, im wesentlichen nicht tagbare Anzeigeeinheit enthält, und daß die Steckverbindungen des weiteren imstande sind, das Modul (20) gleichzeitig mit der tragbaren Anzeigeeinheit (24) und einer der ersten und dritten Anzeigeeinheiten (10) zu verbinden oder individuell das Modul mit einem der Anzeigeeinheiten zu verbinden.

11. Überwachungssystem nach Anspruch 1 oder irgendeinem darauf rückbezogenen Anspruch, dadurch gekennzeichnet, daß die tragbare Anzeigeeinheit (24) imstande ist, Daten in einer Art und Weise anzuzeigen, welche derjenigen der ersten Anzeigeeinheit (10) entspricht.

## Revendications

1. Système de surveillance pour assurer une surveillance continue et ininterrompue d'une ou de plusieurs conditions physiologiques d'un patient, y compris pendant des périodes de transport du patient d'un endroit à un autre, le patient comportant, alors que le système fonctionne, des détecteurs de données de condition physiologique qui lui sont appliqués ; le système comprenant :
une première unité d'affichage relativement non transportable (10) produisant un affichage de données des conditions physiologiques qui sont surveillées ;
un module d'acquisition et de traitement de données (20) pour recevoir des données en provenance des détecteurs ;
le système étant caractérisé par :
une seconde unité d'affichage portable séparée (24) capable d'accompagner le patient pendant son transport et de produire un affichage de données des conditions physiologiques qui sont surveillées ; et
des premier et second connecteurs (42, 44 ; 36, 38, 62) pour coupler le module simultanément aux première et seconde unités d'affichages ou pour coupler le module individuellement à l'une ou l'autre des unités d'affichage, d'où il résulte que le module (20) peut piloter les unités d'affichage (10, 24) pour afficher des données des conditions physiologiques qui sont surveillées.

2. Système de surveillance selon la revendication 1, dans lequel les première (10) et seconde (24) unités d'affichage incluent chacune un moyen (18, 50) pour recevoir le module (20) et dans lequel le module peut être inséré dans l'une ou l'autre des unités d'affichage.

3. Système de surveillance portable (20, 24) pour afficher des données de condition physiologique d'un patient auquel des détecteurs de données de condition physiologique sont appliqués, le système comprenant ;
un module d'acquisition et de traitement de données (20) pour recevoir des données en provenance des détecteurs ; le module comportant un moyen (42) pour connecter le module (20) à une unité d'affichage (10) qui n'est pas destinée à être transportée,
et étant caractérisé en ce que :
une unité d'affichage portable (24) comporte un élément de base (50) comprenant un compartiment et un écran (56) pour afficher des données de condition physiologique ; et
le module d'acquisition et de traitement de données (20) peut être inséré dans le compartiment de l'élément de base (50) de l'unité portable (24), le module comportant :
un premier connecteur (36, 38) pour connecter le module à l'unité portable de telle sorte que le module puisse piloter l'écran (56) afin d'afficher des données de condition physiologique ; et
dans lequel ledit moyen (42) est un second connecteur.

4. Système de surveillance selon l'une quelconque des revendications 1 à 3, dans lequel le second connecteur (36, 38) inclut un cordon de connexion (38) pour coupler le module à l'unité d'affichage portable (24).

5. Système de surveillance selon la revendication 2 ou selon la revendication 4 lorsqu'elle dépend de la revendication 2, dans lequel le premier connecteur inclut un moyen (42) pour connecter le module (20) à la première unité d'affichage (10) lorsque le module est inséré dedans.

6. Système de surveillance selon l'une quelconque des revendications 1 à 5, dans lequel le module (20) contient une alimentation pour alimenter le module et l'unité d'affichage portable (24) lorsque le module lui est couplé.

7. Système de surveillance selon la revendication 1 ou selon l'une quelconque des revendications lorsqu'elle dépend de celle-ci, dans lequel la première unité d'affichage (10) peut être couplée au secteur électrique et dans lequel la première unité d'affichage et le module (20) contiennent un moyen de connexion pour alimenter le module lorsque le module est couplé à la première unité d'affichage.

8. Système de surveillance selon la revendication 2 ou selon la revendication 3, dans lequel l'unité d'affichage portable (24) est formée pour recevoir un module auxiliaires d'acquisition et de traitement de données.

9. Système de surveillance selon l'une quelconque des revendications 1 à 8, dans lequel l'unité d'affichage portable (24) incorpore un affichage à écran plat (56).

10. Système de surveillance selon la revendication 1 ou selon l'une quelconque des revendications lorsqu'elle dépend de celle-ci, incluant en outre une troisième unité d'affichage relativement non transportable et dans lequel les connecteurs sont en outre capables de coupler le module (20) simultanément à l'unité d'affichage portable (24) et à l'une des première et troisième unités d'affichage (10) ou de coupler le module individuellement à l'une des unités d'affichage.

11. Système de surveillance selon la revendication 1 ou selon l'une quelconque des revendications précédentes lorsqu'elle dépend de celle-ci, dans lequel l'unité d'affichage portable (24) est capable d'afficher des données d'une manière qui correspond à celle de la première unité d'affichage (10).
